(19) Europäisches Patentamt
European Patent Office
Office européen des brevets

(11) **EP 3 317 654 B1**

(12) **EUROPEAN PATENT SPECIFICATION**

(45) Date of publication and mention
of the grant of the patent:
**20.01.2021 Bulletin 2021/03**

(51) Int Cl.:
***G01N 27/416*** (2006.01)  ***G01N 27/407*** (2006.01)

(21) Application number: **16818456.2**

(22) Date of filing: **17.06.2016**

(86) International application number:
**PCT/US2016/038007**

(87) International publication number:
**WO 2017/003715 (05.01.2017 Gazette 2017/01)**

(54) **OXYGEN SENSOR FOR CO BREAKTHROUGH MEASUREMENTS**

SAUERSTOFFSENSOR FÜR CO-DURCHBRUCHMESSUNGEN

CAPTEUR D'OXYGÈNE POUR DES MESURES DE PÉNÉTRATION DE CO

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2015 US 201514788203**

(43) Date of publication of application:
**09.05.2018 Bulletin 2018/19**

(73) Proprietor: **Rosemount Inc.
Solon OH 44139 (US)**

(72) Inventors:
• **SHUK, Pavel
Copley, OH 44321 (US)**
• **JANTZ, Robert, F.
Tustin, CA 92782 (US)**
• **KRAMER, James, D.
Homerville, OH 44235 (US)**

(74) Representative: **Parker, Andrew James
Meissner Bolte Patentanwälte
Rechtsanwälte Partnerschaft mbB
Postfach 86 06 24
81633 München (DE)**

(56) References cited:
**EP-A2- 1 327 880     GB-A- 1 523 214
JP-A- 2003 222 607     JP-B2- 3 435 836
US-A- 5 795 545     US-A1- 2004 244 363
US-A1- 2006 236 752     US-A1- 2014 338 422
US-B1- 7 414 726     US-B1- 7 886 523**

• **JAUME FOLCH ET AL: "Solid Electrolyte Multisensor System for Detecting O2, CO, and NO2", JOURNAL OF THE ELECTROCHEMICAL SOCIETY, vol. 154, no. 7, 11 May 2007 (2007-05-11), page J201, XP055517677, ISSN: 0013-4651, DOI: 10.1149/1.2735926**
• **CAN Z Y ET AL: "Detection of carbon monoxide by using zirconia oxygen sensor", SOLID STATE IONICS, NORTH HOLLAND PUB. COMPANY. AMSTERDAM; NL, NL, vol. 79, 2 July 1995 (1995-07-02), pages 344-348, XP004050366, ISSN: 0167-2738, DOI: 10.1016/0167-2738(95)00085-K**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND

The process industries often rely on energy sources that include one or more combustion processes. Such combustion processes include operation of a furnace or boiler to generate steam or to heat a feedstock liquid. While combustion provides relatively low cost energy, combustion efficiency is sought to be maximized. In addition, flue gases from industrial processes exiting smokestacks are often regulated, and the amount of dangerous gases often must be minimized. Accordingly, one goal of the combustion process management industry is to maximize combustion efficiency of existing furnaces and boilers, which inherently also reduces the production of greenhouse and other regulated gases. Combustion efficiency can be optimized by maintaining the ideal level of oxygen in the exhaust or flue gases coming from such combustion processes.

In-situ or in-process analyzers are commonly used for the monitoring, optimization, and control of the combustion process. Typically, these analyzers employ sensors that are heated to relatively high temperatures and are operated directly above, or near, the furnace or boiler combustion zone. Known process combustion oxygen analyzers typically employ a zirconium oxide sensor disposed at an end of a probe that is inserted directly into a flue gas stream. As the exhaust, or flue gas, flows into the sensor, it diffuses into proximity with the sensor. The sensor provides an electrical signal related to the amount of oxygen present in the gas. Sensor systems for detecting oxygen and carbon monoxide are e.g. described by Folch et al. (2007), in "Solid Electrolyte Multisensor System for Detecting O2, CO, and NO2", Journal Of The Electrochemical Society, Vol. 1046, p. J201; Can et al. (1995), in "Detection of carbon monoxide by using zirconia oxygen sensor", Solid State Ionics, p.344-348; GB 1523214 A and EP 1327880 A2.

SUMMARY

A sensor system configured to detect oxygen in an exhaust stream of an industrial process is provided, as defined by independent claim 1. A corresponding method for detecting carbon monoxide in an exhaust stream of an industrial process is defined by independent claim 8. In one embodiment, the sensor system comprises a probe with an oxygen-detecting sensor, wherein the oxygen-detecting sensor detects a concentration of oxygen in the exhaust stream. The system may also comprise a catalytic converter located on the probe near the sensor, wherein the catalytic converter is configured to convert carbon monoxide to carbon dioxide. The system may also comprise a signal detector configured to comprise two detection modes, wherein the signal detector is configured in the first mode to detect a change in oxygen concentration indicative of a carbon monoxide breakthrough based on the signal from the oxygen-detecting sensor, and in the second mode to detect a change in oxygen concentration indicative of a carbon monoxide breakthrough based on a derivative of an oxygen sensor signal response. These and various other features and advantages that characterize the claimed embodiments will become apparent upon reading the following detailed description and upon reviewing the associated drawings.

BRIEF DESCRIPTION OF THE DRAWINGS

FIG. 1 is a diagrammatic view of an in-situ process oxygen analyzer/transmitter with which embodiments of the present invention are particularly applicable.
FIG. 2 is a diagrammatic perspective view of a combustion oxygen transmitter with which embodiments of the present invention are particular applicable.
FIGS. 3A - 3E are graphical representations of CO measurements using an oxygen sensor in accordance with an embodiment of present invention.
FIGS. 4A - 4D are graphical representations of an oxygen sensor response to carbon monoxide in accordance with an embodiment of present invention.
FIGS. 5A and 5B illustrate exemplary methods of measuring the concentration of carbon monoxide utilizing an oxygen sensor in accordance with an embodiment of present invention.

DETAILED DESCRIPTION

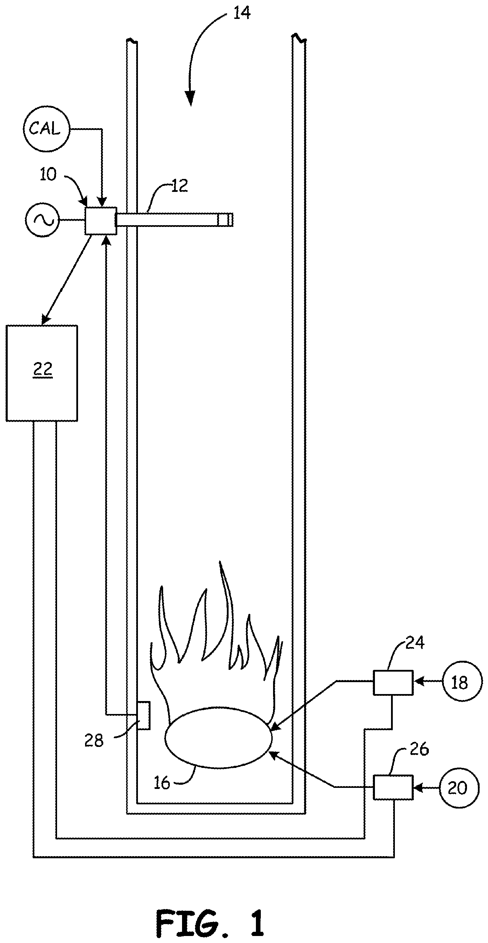

FIG. 1 is a diagrammatic view of an in-situ process oxygen analyzer transmitter installation with which embodiments of the present invention are particularly applicable. Transmitter 10 can be, for example, a Model 6888 oxygen transmitter available from Rosemount Analytical Inc., of Solon, Ohio (an Emerson Process Management company). Transmitter 10, in one embodiment, includes probe assembly 12 that is substantially disposed within stack or flue 14 and measures oxygen content of the flue gas related to combustion occurring at burner 16. In one embodiment, burner 16 is operably coupled to a source of air or oxygen source 18 and source 20 of combustion fuel. Each of sources 18 and 20 are controllably coupled to burner 16 in order to control the combustion process, in one embodiment. Transmitter 10 measures the amount of oxygen in the combustion exhaust flow and provides an indication of the oxygen level to combustion controller 22, in one embodiment. Controller 22 controls one or both of valves 24 and 26 to provide closed loop combustion control. In one embodiment, controller 22 operates automatically, such that an indication of too much or too little oxygen in the exhaust flow results in a change in the amount of oxygen or fuel provided to the combustion chamber. In one example, the oxygen analyzer transmitter may also include a calibration line with calibration gases 28, connected to transmitter 10.

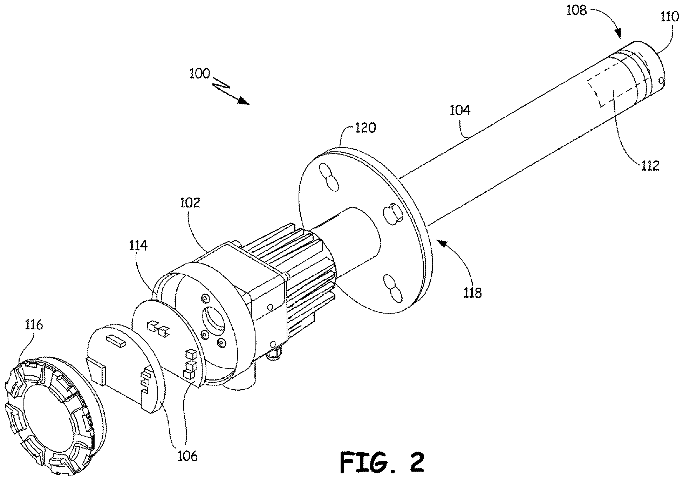

FIG. 2 is a diagrammatic perspective view of a combustion oxygen transmitter with which embodiments of the present invention are particularly applicable. Transmitter 100 includes housing 102, probe body 104, and electronics 106 with a protective cover 116. Probe 104 has a distal end 108 where a diffuser 110 is mounted. The diffuser 110 is a physical device that allows gaseous diffusion therethrough, but otherwise protects components within probe 104 from solid particles like fly ash. Specifically, diffuser 110 protects from the dust a measurement cell, or sensor 112, illustrated in phantom in FIG. 2.

Housing 102 has a chamber 114 that is sized to house electronics 106. Additionally, housing 102 includes internal threads that are adapted to receive and mate with external threads of cover 116 to make a hermetic seal. Additionally, housing 102 includes a bore or aperture therethrough allowing electrical interconnection between electronics 106 and measuring cell or sensor 112 disposed within distal end 108 of the probe 104.

Probe 104 is configured to extend within a flue, such as flue 14. Probe 104 includes a proximal end 118 that is adjacent to flange 120. Flange 120 is used to mount or otherwise secure the transmitter 100 to the sidewall of the duct. When so mounted, transmitter 100 may be completely supported by the coupling of flange 120 to the duct wall.

Electronics 106 provide heater control and signal conditioning, resulting in a linear 4-20 mA signal representing flue gas oxygen concentration. Preferably, electronics 106 also includes a microprocessor that is able to execute programmatic steps to provide the functions of diffuser diagnostics. However, in some embodiments, transmitter 100 may simply be a "direct replacement" probe with no electronics and thus sending raw millivolt signals for the sensing cell and thermocouple providing indications representative of the oxygen concentration and cell temperature respectively. In embodiments where a "direct replacement" probe is used, the probe is coupled to a suitable analyzer such as the known Xi Operator Interface available from Rosemount Analytical Inc. The Xi Operator Interface provides a backlit display, signal conditioning and heater control within a NEMA 4X (IP 66) housing.

Ideally, combustion in an industrial process is perfect, and fuel and oxygen combust to create carbon dioxide and water, according to Equation 1 below, where the stoichiometric amount of carbon dioxide and water produced is dependent on the type of fuel used in a specific industrial process.

$$Fuel + O_2 \rightarrow CO_2 + H_2O \hspace{4cm} \text{Equation 1}$$

Often, however, combustion in industrial processes is not perfect and, in addition to carbon dioxide and water, excess oxygen exists in the exhaust. In one embodiment, while the industrial process is in a regular operating mode, an oxygen sensor, for example, oxygen transmitter 100 with probe 104, measures the remaining oxygen gas in the exhaust of a the combustion process. Additionally, as is often the case, incomplete combustion occurs according to Equation 2 below.

$$Fuel + O_2 \rightarrow CO_2 + H_2O + (CO + NO_x + SO_x)_{incomplete\ products} \hspace{1cm} \text{Equation 2}$$

In an imperfect combustion, fuel comes into the industrial process with some contaminants and reacts to form, primarily carbon dioxide, $CO_2$, and water $H_2O$, with traces of other gases such as sulfur dioxide, nitrogen oxides, which come from the fuel impurities, as well as nitrogen oxidation. Additionally, when insufficient oxygen is provided to the industrial process, carbon monoxide forms as part of the incomplete combustion. The stoichiometric point, e.g. the ratio of fuel to oxygen with the highest efficiency and lowest emissions is very difficult to achieve in real combustion because of imperfect fuel to air uniformity, and of the fuel energy density and fuel to air flow variation.

Typically, flue gas oxygen excess concentration is 2-3 percent for gas burners and 2-6 percent for coal fired boilers and oil burners. The most efficient combustion may occur, in one embodiment, between 0.75 percent and 2 percent oxygen excess. While good combustion control can be accomplished with oxygen measurement alone, combustion efficiency and stability can be improved with the concurrent measurement of carbon monoxide, CO. As shown above in Equation 2, carbon monoxide is often a result of incomplete combustion of the fuel and oxygen supply and, therefore, a good first indicator that incomplete combustion is occurring in the process.

The development of carbon monoxide often occurs when the oxygen level is below a required amount for the industrial process to complete Equation 1 above. As carbon monoxide is a dangerous by-product of an incomplete combustion, its presence in exhaust gas may be regulated and an industrial process may be designed to include a catalytic converter to allow for conversion of carbon monoxide to carbon dioxide, according to Equation 3 below.

$$CO + \frac{1}{2}O_2 \longleftrightarrow CO_2 \qquad\qquad \text{Equation 3}$$

In one embodiment, the excess oxygen is sampled periodically by probe 104 throughout combustion. In one embodiment, the excess oxygen is sampled by probe 104 almost continually throughout a combustion process. The oxygen sensor output may be reported on an attached display, in one embodiment. The oxygen sensor output may also be transmitted to a database for storage. In another embodiment, the oxygen sensor output may be attached to an alarm system wherein certain maximum or minimum threshold oxygen concentrations may trigger a process alarm or an alert to a process engineer that a threshold has been surpassed. In one embodiment, the alert may be sent through text message, e-mail or another wireless-based delivery mechanism. In another embodiment, the alert may be an audiovisual alert within the industrial process, and may result in either a light being activated, a sound being emitted, or a combination of alert mechanisms that a threshold has been surpassed.

In another embodiment, oxygen transmitter 100 is coupled to controllers 24 and 26 such that readings from the oxygen transmitter can trigger automatic changes in the ratio of oxygen to fuel entering the industrial process. For example, upon a reading indicative of a rich exhaust mixture, indicating a high amount of unburned fuel and low remaining oxygen, the transmitter 100 may trigger controller 24, allowing more oxygen into the system, and / or may also trigger controller 26 inputting a lower amount of fuel into the system. The system may be calibrated, in one embodiment, to automatically adjust controllers 24 and 26 until a lean mixture is achieved. In one embodiment, a lean mixture is defined as a mixture of fuel and oxygen sufficient to convert the fuel into water and carbon dioxide without any incomplete combustion products.

In one embodiment, transmitter 100 is based on electrochemical zirconia-based cell technology. In one embodiment, the probe 104 is based on a solid-state electrochemical cell consisting of at least one zirconia ceramic located between an exhaust gas sample on one side, and a reference sample on the other side, wherein gas permeable electrodes are located on either side of the zirconia ceramic. The zirconia-based sensor 104 measures a concentration of remaining oxygen in the exhaust gas by measuring an output voltage across the zirconia ceramic, corresponding to a quantity of oxygen in the exhaust of the industrial process measured against a quantity of oxygen in a reference sample. The voltage measured corresponds to a concentration differential of oxygen between the two samples and, therefore, to an amount of oxygen consumed in a combustion reaction according to Equation 1 above. In one embodiment, the reference sample contains air of substantially atmospheric quality.

The oxygen sensor readings may depend logarithmically on the oxygen concentration according to the Nernst equation, Equation 4, below.

$$EMF = \frac{RT}{4F} ln\left(\frac{P_{process}}{P_{ref}}\right) + C = 0.0496 \times T \times log\left(\frac{P_{process}}{P_{ref}}\right) + C \qquad \text{Equation 4}$$

Zirconia based electrochemical oxygen sensors are widely used in industrial applications for oxygen measurements. In one example, the sensor 104 works at temperatures in the 650-800 degree C ranges and above, and measures the excess oxygen remaining after combustion. The response of the sensor to the differential oxygen concentration with a fixed oxygen partial pressure on the reference electrode, for example, fixed by using air can be calculated by using equation 4 above. In Equation 4, C is the constant related to the reference/process side temperature variation and thermal junctions in the oxygen probe, R is the universal gas constant, T is the process temperature, measured in degree Kelvin, and F is the Faraday constant.

In the combustion process, carbon monoxide is often the first indicator of an incomplete combustion. Operation at near trace CO levels of about 100 to 200 ppm and a slight amount of excess air would indicate the combustion conditions near the stoichiometric point with the highest efficiency. While there are many CO sensors available for applications ranging from workspace safety to exhaust gas analysis, the high temperature of typical industrial processes presents a difficulty in providing a reliable in-situ CO measurement for a combustion process.

A number of studies have been done on chemical gas sensors based on semiconducting oxides that are now used worldwide for combustible gas detection. This type of sensor, known as the Taguchi sensor, employs a solid state device made of sintered n-type metallic oxide (iron, zinc, and tin families), but poor selectivity and insufficient long term stability have been the major difficulties of these semiconducting sensors in the process environment.

Infrared absorption techniques relying on measuring the infrared light absorption would mostly require the flue gas conditioning system and thus add a relatively large expense to an industrial process. New, very sophisticated, and highly promoted tunable diode laser spectroscopy uses much more powerful laser light, is more reliable, and does not require a flue gas preconditioning. Unfortunately, fouling at a heavy particulate load, wide background radiation from the fireball, and required temperature and pressure compensation, as well as very high price, limit this technology to the applications in the chemical industry and for applications requiring high temperatures, such as combustion related processes. Currently, the only in-situ CO probe available on the market, and based on mixed potential zirconia technology was developed for very clean gas combustion application.

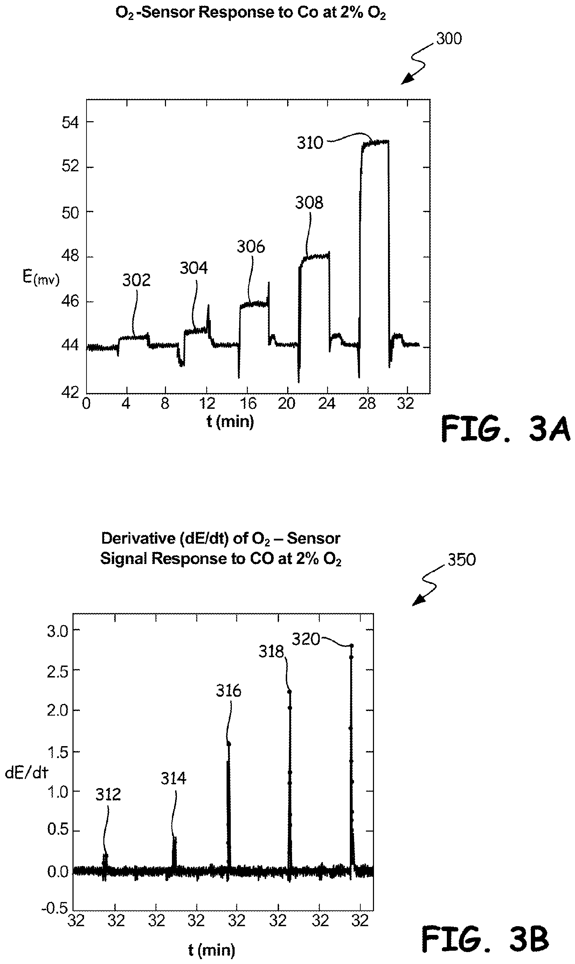

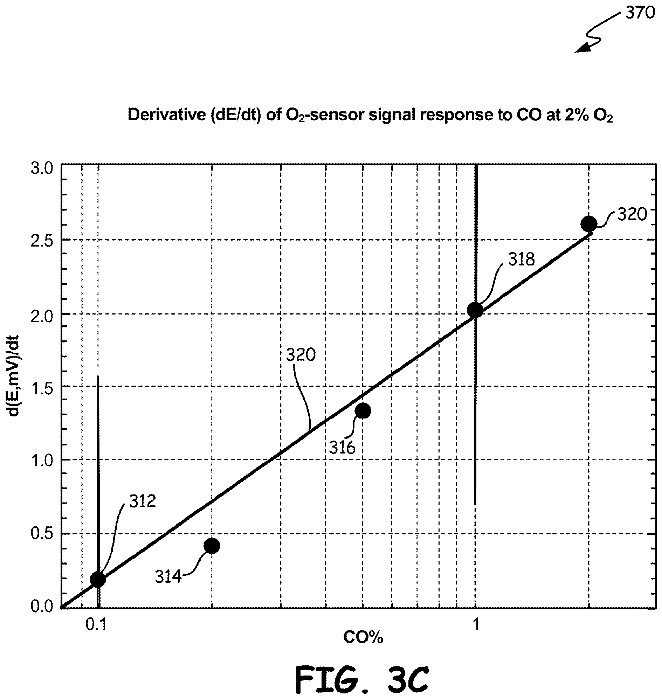

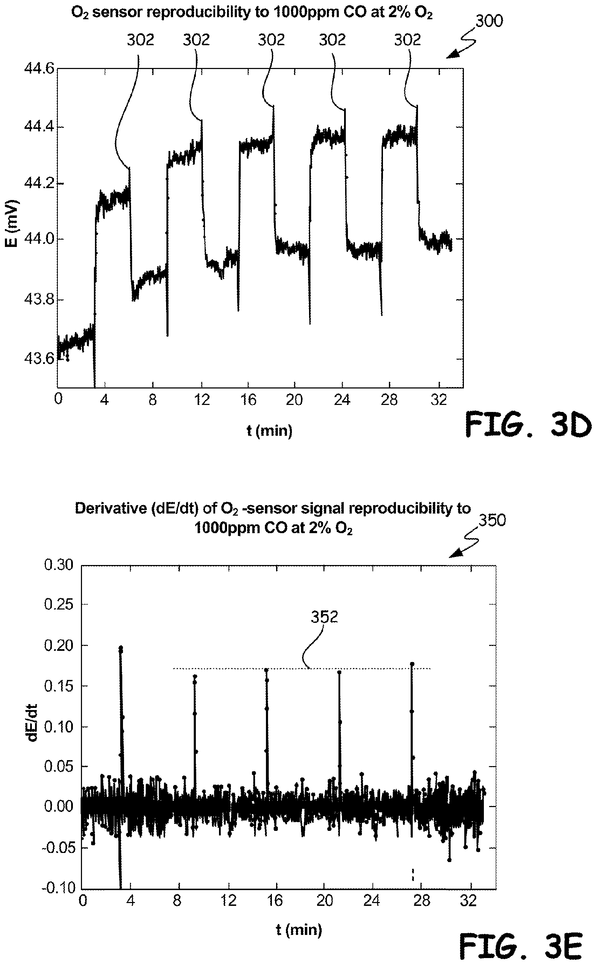

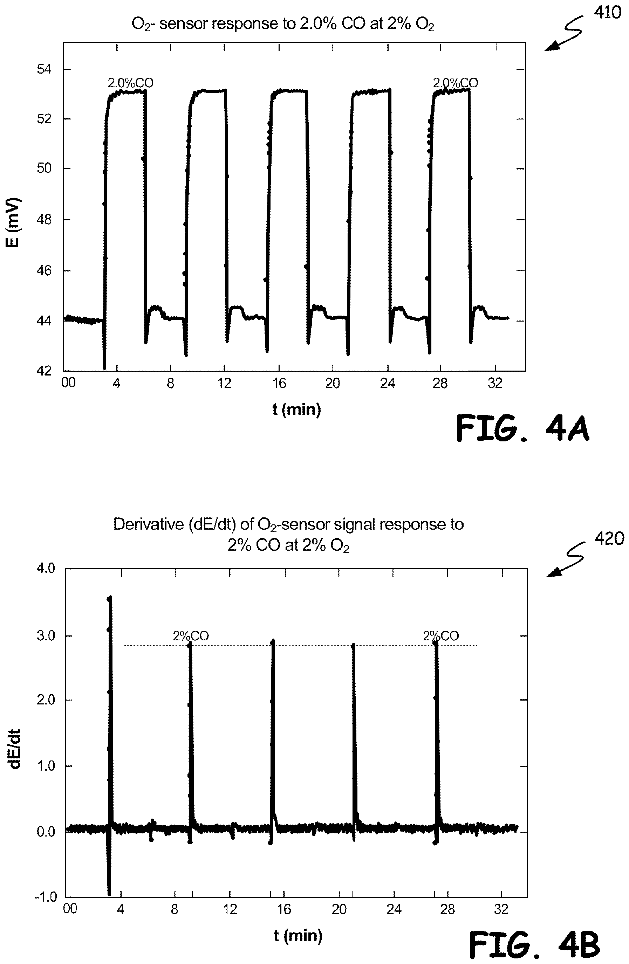

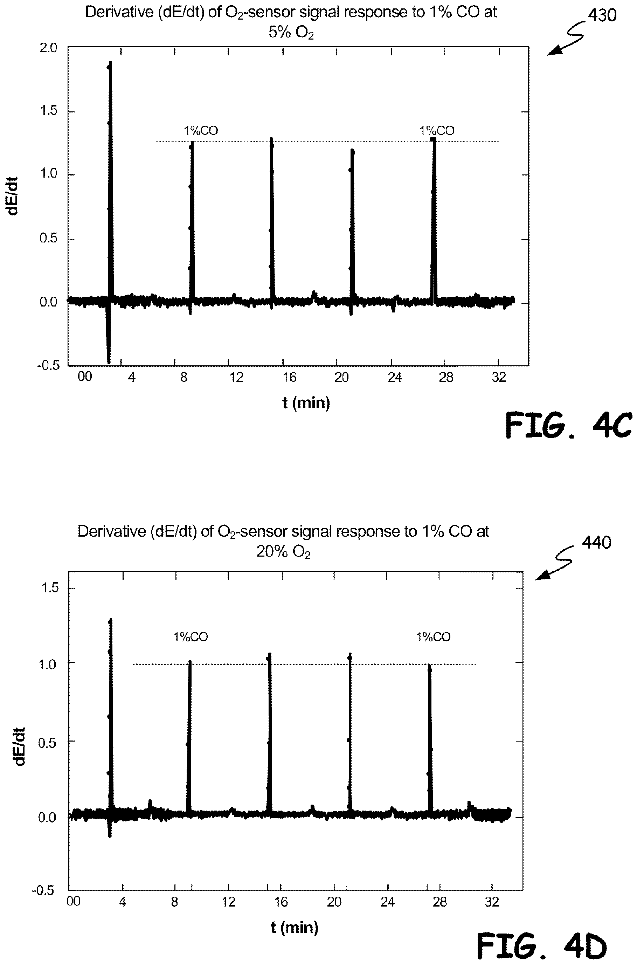

In one example, the solid state potentiometric gas sensor for the oxygen measurements in the process comprises an oxide ion conducting ceramic in the form of a tube, disc or thimble, and two metallic or oxide catalytic electrodes that are exposed to the process and reference gases, respectively. In one embodiment, the ionic conducting ceramic is mostly a doped zirconia but could be stabilized cerium or bismuth oxide or any other oxide ion conducting solid electrolyte. The process reference electrodes are in one example platinum, but any other electron conducting metal or metal oxide or mixed conducting pure or composite material could also be used. The oxygen sensor's process electrode is exposed to the flue gas, and the oxygen sensor is in an oxidizing environment in the regular potentiometric mode precisely measuring excess oxygen concentration in the combustion process flue gas. The highest peak of the derivative of the oxygen sensor signal is used as an additional carbon monoxide sensing output. The oxygen sensor, in one embodiment, is calibrated using fixed CO concentrations. This may correlate, for example, to the results shown in FIGS. 3 and 4. The measured oxygen sensor raw mV signal is used to calculate an oxygen concentration according to equation 4, the Nernst equation, and the highest peak of the derivative of the oxygen sensor signal is applied for carbon monoxide breakthrough calculation using developed carbon monoxide algorithm validated with the carbon monoxide calibration gases. An additional oxygen sensor signal noise reduction, could be applied, in one example, to remove the parasite electrical spikes in the industrial application that may alter oxygen and carbon monoxide measurements.

OXYGEN SENSOR DETECTING CARBON MONOXIDE

Carbon monoxide is known to be one of the first products of an incomplete combustion to appear in a process. The presence of carbon monoxide results in a decrease in oxygen concentration as the carbon monoxide breaking through in the combustion process will, in one embodiment, be immediately converted to carbon dioxide, according to Equation 3 above, on a platinum electrode catalyst that is located on the sensor 112. In one example, the platinum electrode catalyst is located very close to the oxygen-sensing portion of sensor 112. This will result in the oxygen concentration being significantly reduced near the oxygen-sensing electrochemical cell, as a result of the catalytic conversion of carbon monoxide to carbon dioxide, resulting in a sudden increase in the raw mV signal produced by sensor 112. This will result in the oxygen sensor output signal indicating an immediate reduction in oxygen concentration, especially in the milliseconds after carbon monoxide breakthrough in a combustion scenario. This may trigger, as indicated above, an alert provided to a process engineer, or it may trigger a change in the ratio of fuel and oxygen sources 20 and 18, respectively, through alteration of controls 26 and 24, respectively.

The detection of a drop in concentration of oxygen gas in the exhaust provides a quantitative indication of carbon monoxide that was present in the exhaust gas prior to the conversion and, therefore, an indication of the concentration of carbon monoxide produced as a result of the combustion. As can be seen from Table 1 below, CO presence is reducing the oxygen signal by almost 50 percent of the CO concentration, with CO conversion rate varying between 80 to 100% at 1000 ppm CO to 60 to 100% at 2% CO.

TABLE 1: OXYGEN SENSOR PROBE RAW mV SIGNAL REDUCTION

| $[O_2]$ (%) | [CO] (%) | | | | | | | |
|---|---|---|---|---|---|---|---|---|
| | 0.1 | | 0.5 | | 1.0 | | 2.0 | |
| | $\Delta E_t$ (mV) | $\Delta E_m$ (mV) | $\Delta E_t$ (mV) | $\Delta E_m$ (mV) | $\Delta E_t$ (mV) | $\Delta E_m$ (mV) | $\Delta E_t$ (mV) | $\Delta E_m$ (mV) |
| 2 | 0.55 | 0.43 | 2.9 | 1.8 | 6.3 | 3.9 | 15.1 | 9.1 |
| 3 | 0.36 | 0.30 | 1.9 | 1.3 | 4.0 | 2.8 | 8.8 | 6.4 |
| 4 | 0.25 | 0.24 | 1.4 | 1.0 | 2.9 | 2.2 | 6.3 | 4.9 |
| 5 | 0.22 | 0.20 | 1.1 | 0.8 | 2.3 | 1.8 | 4.9 | 3.9 |
| 20.9 | 0.05 | 0.05 | 0.26 | 0.26 | 0.53 | 0.52 | 1.06 | 1.05 |

In Table 1 the change in sensor signal is theoretical $\Delta E_t$ and calculated assuming 100% CO combustion by the platinum catalytic converter. The measured signal change $\Delta E_m$ being close to theoretical change $\Delta E_t$, Table 1 does prove the effectiveness of an oxygen sensor, such as probe 112, to detect carbon monoxide in an industrial environment.

EXAMPLES OF CARBON MONOXIDE DETECTION WITH AN OXYGEN SENSOR

FIGS. 3A - 3E are graphical representations of CO measurements using an oxygen sensor in accordance with an embodiment of the present invention. Specifically, FIGS. 3A - 3E illustrate the responses of an oxygen sensor 112 to the presence of carbon monoxide at various levels of oxygen over time in an industrial process. More specifically, FIGS. 3A and 3D illustrate a direct sensor response. FIGS 3B, 3C and 3E illustrate the derivative of the oxygen sensor signal response to carbon dioxide.

FIG. 3A illustrates the oxygen sensor 112 response to carbon monoxide in an environment with a two percent oxygen concentration over a period of time, with time on the X axis, and the response of the oxygen sensor, in mV, shown on the Y axis. At around four minutes, a spike 302 is shown that corresponds to roughly 1000 ppm presence of carbon monoxide in the industrial process, as indicated by a reading of approximately 44.5 mV. At around ten minutes, a spike 304 corresponds to a 2000 ppm presence of carbon monoxide, as indicated by a reading of just under 45 mV. At around fifteen minutes, a spike 306, corresponding to a 0.5% carbon monoxide concentration, results in a reading of approximately 46 mV. At around twenty one minutes, a spike of one percent carbon monoxide is detected, as indicated by a reading of approximately 48 mV. At around twenty seven minutes, a spike 310 is detected, indicating a two percent presence of carbon monoxide, with a corresponding reading of 53 mV by the sensor 112. As can be seen from FIG. 3A, oxygen sensor raw mV signal is highly sensitive to carbon monoxide gas presence at 2% oxygen concentration, with a 9 mV change in sensor reading detected.

The sensor signal derivative over time, as shown in FIG. 3B, is also presented, with time on the x-axis and the derivative value on the y-axis. The highest peak value of the derivative dE/dt is logarithmically dependent on the carbon monoxide concentration in the range between 1000 ppm and 2% carbon monoxide. At approximately 4 minutes, a spike 312 is shown, corresponding to a concentration of approximately 1000 ppm carbon monoxide, with a reading of approximately 0.2. At approximately ten minutes, spike 314 indicates a carbon monoxide concentration of approximately 2000 ppm, resulting in a reading of approximately 1.0 by the sensor 112. At approximately fifteen minutes, spike 316 indicates a carbon monoxide concentration of 0.5%, resulting in a reading of approximately 1.5 by the sensor 112. At approximately twenty-one minutes, spike 318 indicates a carbon monoxide concentration of approximately 1% carbon monoxide, resulting in a reading of approximately 2.2 by the sensor 112. At approximately twenty-seven minutes, spike 320 indicates a carbon monoxide concentration of 2%, with a reading of approximately 2.7 by the sensor 112.

In one example, sensor 112 outputs the raw mV data graphically, as shown in FIG. 3A. In another example, sensor 112 outputs the derivative dE/dt value graphically, as shown in FIG. 3B. In an preferred embodiment, sensor 112 outputs a current carbon monoxide concentration, calculated from either the data obtained from the sensor, for example as shown in FIG. 3A or FIG. 3B. In another embodiment, detection of a minimum threshold of carbon monoxide concentration in the exhaust gas triggers a change in the ratio of fuel to oxygen input into the industrial process. In another embodiment, detection of a minimum threshold concentration of carbon monoxide in the exhaust gas triggers an alert.

FIG. 3C illustrates a graphical representation 370 of the derivative of the $O_2$ sensor signal response shown in FIG. 3B, with the carbon monoxide concentration presented logarithmically on the x-axis, and the dE/dt values obtained on the y-axis. Equation 5, shown below, is also shown as line 320. Equation 5 has an R value of 0.9912.

$$\frac{dE}{dt} = 1.97 + 1.948 \times \log[\% \, CO] \qquad \text{Equation 5}$$

FIG. 3D illustrates the reproducibility of the oxygen sensor 112 to repeated carbon monoxide breakthroughs of 1000 ppm, in an environment with 2% oxygen. Indications 302 are shown as occurring roughly at four minutes, nine minutes, sixteen minutes, twenty one minutes and twenty-seven minutes. As shown in FIG. 3D, the raw mV value of oxygen sensor 112 may exhibit slight drifting in the response to the presence of 1000 ppm carbon monoxide.

FIG. 3E illustrates the increased reproducibility of detected carbon monoxide using the derivative of the oxygen sensor signal, with repeated 1000 ppm carbon monoxide breakthroughs occurring at nine, fifteen, twenty one and twenty seven minutes all resulting in a detected reading of approximately 0.17, as represented by bar 352. Additionally, the derivative of the oxygen sensor signal highest peak is shown at 1000 ppm carbon monoxide with about a 70 ppm carbon monoxide error as shown in FIG. 3E.

FIGS. 4A - 4D are graphical representations of an oxygen sensor response to carbon monoxide. FIG. 4A illustrates a graph 410 showing the reproducibility of detecting a 2% carbon monoxide concentration, with peaks at approximately four, nine, fifteen, twenty one and twenty seven minutes all resulting in a detected reading of approximately 52 mV. FIG. 4B illustrates a graph 420 showing the reproducibility of the derivative of the oxygen sensor response at detecting a 2% carbon monoxide concentration, with peaks at approximately four, nine, fifteen, twenty one and twenty seven minutes all resulting in a detected reading of approximately 2.8, with an error rate in detection of approximately $\pm 0.02\%$ CO, or a 1% error rate. FIG. 4C illustrates a graph 430 showing the reproducibility of the derivative of the oxygen sensor response at detecting a 1% carbon monoxide concentration in a 5% oxygen environment, with peaks at approximately nine, fifteen, twenty one and twenty seven minutes all resulting in a detected reading of approximately 1.3. FIG. 4D illustrates a graph 440 showing the reproducibility of the derivative of the oxygen sensor response at detecting a 1% carbon monoxide concentration in a 20% oxygen environment, with peaks at approximately four, nine, fifteen, twenty one and twenty seven minutes all resulting in a detected reading of approximately 1.0.

Thus, FIGS. 3A-3E and FIGS 4A-4D show that, through the use of a zirconia electrochemical oxygen sensor, and the derivative of the sensor signal response, reliable carbon monoxide measurements can be provided in a variety of carbon monoxide breakthrough scenarios.

METHODS OF DETECTING CARBON MONOXIDE

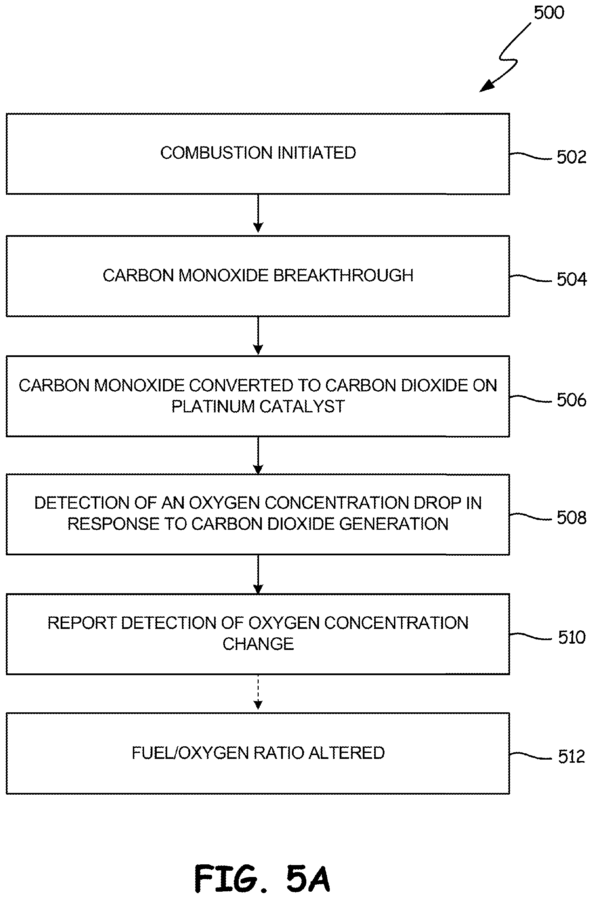

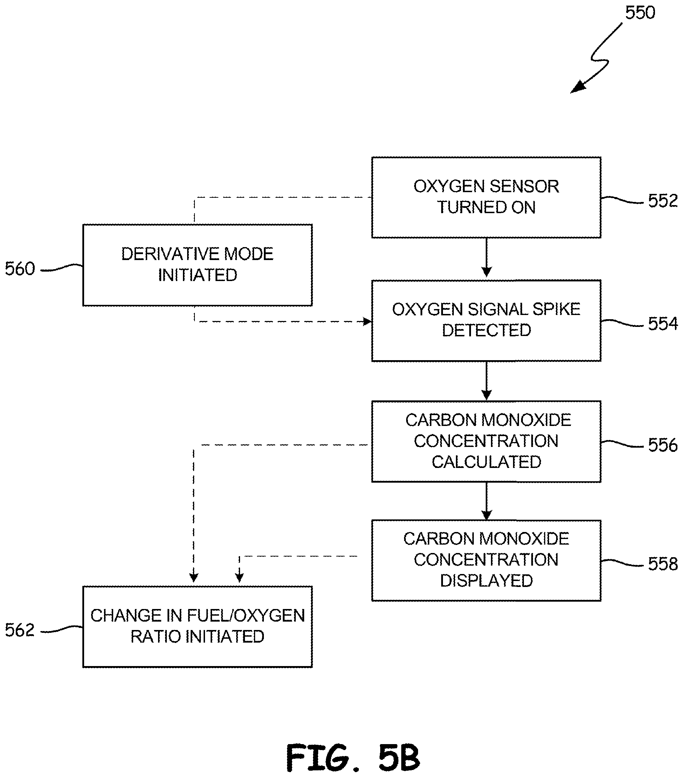

FIGS. 5A and 5B illustrate exemplary methods of measuring development of carbon monoxide utilizing an oxygen sensor. FIG. 5A illustrates an exemplary method 500 of detecting and displaying a carbon monoxide breakthrough using an oxygen sensor. In one embodiment, the oxygen sensor may include the zirconia-based electrochemical cell described above.

Method 500 starts in block 502 with a combustion initiating in an industrial process. Method 500 continues with a carbon monoxide breakthrough occurring as shown in block 504. In one example it may be minutes, hours, or longer between a combustion process starting and a carbon monoxide breakthrough occurring. Method 500 continues, in one embodiment, with the produced carbon monoxide being converted to carbon dioxide on a catalyst. In one embodiment, the catalyst is a platinum-based catalyst. In one example this occurs as described above with respect to Equation 3. As the carbon monoxide is converted to carbon dioxide, the measured oxygen concentration in the exhaust gas drops. This drop is detected in block 508 by the probe 104. In one embodiment, the detection is reported in block 510. In one embodiment, as illustrated by method 500, a concentration of carbon monoxide is not calculated as part of the detection process. In an optional embodiment, the method 500 moves on to block 512, where the fuel to oxygen input ratio is altered. This alteration may happen, in one embodiment, automatically, upon the detection of carbon monoxide. In one embodiment, this may result in additional air or oxygen being input to the system through source 18. In one embodiment, this may result in reduced fuel being input to the system through source 20.

FIG. 5B illustrates a method 550 for calculating the carbon monoxide concentration in an exhaust stream using oxygen sensor 112. Method 550 starts in block 552 with the oxygen sensor 112 provided to an industrial process environment. Then, in block 554, an oxygen signal spike is detected, for example, any oxygen signal spike is shown in any of FIGS. 3 and 4. In one example, the sensor 112 operates in a normal, and not a derivative sensing mode. In an embodiment, sensor 112 operates in either a normal or a derivative sensing mode, and the derivative sensing mode is optionally initiated in block 560. In another example, the oxygen sensor may detect both an oxygen sensor signal response and a derivative of the oxygen sensor signal response. However, in another example the sensor may only detect the derivative of the oxygen sensor signal response.

The method then moves on to block 556 wherein a carbon monoxide concentration is calculated based at least in part on Equation 4, described above. The method may then continue, in one embodiment to block 558 where the detected carbon monoxide concentration is displayed, for example, on a connected computer or other display device. Additionally, in another embodiment, displaying the carbon monoxide concentration may comprise sending an alert to a process engineer, for example using wireless or other technology. This may trigger an indication to an operator of the industrial process that there has been a carbon monoxide breakthrough and that fuel to air ratios may need to be changed. The alert could be triggered visually, audibly, or through another means of notification. Additionally, in another embodiment, detection of a carbon monoxide breakthrough may result in an automatic change in the fuel to oxygen ratio, as indicated in block 562.

**Claims**

1. A sensor system configured to detect oxygen in an exhaust stream of an industrial process, the sensor system comprising:

    a probe (104) with an oxygen-detecting sensor (112), wherein the oxygen-detecting sensor (112) is configured to detect a concentration of oxygen in the exhaust is configured to output stream and a signal corresponding, at least in part, to the detected concentration of oxygen;

a catalytic converter located on the sensor (112), wherein the catalytic converter is configured to convert carbon monoxide to carbon dioxide; and

a signal detector, **characterized in that** the signal detector is configured to comprise two detection modes, wherein the signal detector is configured in the first mode to detect a change in oxygen concentration indicative of a carbon monoxide breakthrough based on the signal from the oxygen-detecting sensor, and in the second mode to detect a change in oxygen concentration indicative of a carbon monoxide breakthrough based on a derivative of an oxygen sensor signal response.

2.  The sensor system of claim 1, wherein detection of the carbon monoxide breakthrough triggers an alert.

3.  The sensor system of claim 1, wherein detection of the carbon monoxide breakthrough triggers a change in a current fuel to oxygen ratio input to the industrial process.

4.  The sensor system of claim 1, and further comprising:
    a transmitter (100) configured to transmit an indication of the detected carbon monoxide breakthrough.

5.  The sensor system of claim 1, wherein the oxygen-detecting sensor (112) comprises an electrochemical zirconia-based cell, and wherein the electrochemical zirconia-based cell measures a voltage across electrodes separating a reference gas from the exhaust stream.

6.  The sensor system of claim 1, wherein the catalytic converter comprises a platinum catalyst.

7.  The sensor system of claim 1, wherein the signal detector is further configured to calculate a concentration of carbon monoxide in the exhaust stream, at least based in part on the detected change in oxygen concentration.

8.  A method for detecting carbon monoxide in an exhaust stream of an industrial process, the method comprising:
    detecting an abrupt change in oxygen concentration in the exhaust stream of the industrial process, wherein the detected abrupt change is indicative of a carbon monoxide breakthrough and wherein the detection is completed utilizing an oxygen sensor (112) and its oxygen sensor signal; **characterized by** calculating, in a first detection mode, a concentration of carbon monoxide in the exhaust stream using the detected change in oxygen concentration based on the oxygen sensor signal; and calculating, in a second mode, a concentration of carbon monoxide in the exhaust stream using the detected change in oxygen concentration based on a derivative of an oxygen sensor signal response.

9.  The method of claim 8, and further comprising:
    reporting the detected change in oxygen concentration.

10. The method of claim 8, and further comprising:
    reporting the calculated change in carbon monoxide concentration.

11. The method of claim 8, and further comprising:
    altering an existing fuel-to-oxygen ratio based at least in part on the detected concentration of carbon monoxide to a new fuel-to-oxygen ratio.

12. The method of claim 8, and further comprising:
    transmitting an indication of the calculated carbon monoxide concentration.

13. The method of claim 8, and further comprising:
    triggering an alert indicating that carbon monoxide is present within the exhaust stream of the industrial process.

14. The method of claim 8, wherein the oxygen sensor (112) comprises a zirconia-based electrochemical cell.


**Patentansprüche**

1.  Sensorsystem, das konfiguriert ist, um Sauerstoff in einem Abgasstrom eines industriellen Prozesses nachzuweisen, wobei das Sensorsystem umfasst:

eine Sonde (104) mit einem Sauerstoffdetektionssensor (112), wobei der Sauerstoffdetektionssensor (112) konfiguriert ist, um eine Sauerstoffkonzentration im Abgasstrom nachzuweisen, und konfiguriert ist, um ein Signal auszugeben, das zumindest teilweise der nachgewiesenen Sauerstoffkonzentration entspricht;

einen am Sensor (112) angeordneten katalytischen Konverter, wobei der katalytische Konverter konfiguriert ist, um Kohlenmonoxid in Kohlendioxid umzuwandeln; und

einen Signaldetektor, **dadurch gekennzeichnet, dass**

der Signaldetektor konfiguriert ist, um zwei Erfassungsmodi zu umfassen, wobei der Signaldetektor im ersten Modus konfiguriert ist, um auf Basis des Signals von dem Sauerstoffdetektionssensor eine Änderung der Sauerstoffkonzentration nachzuweisen, die auf einen Kohlenmonoxid-Durchbruch hinweist, und im zweiten Modus konfiguriert ist, um auf Basis einer Ableitung einer Sauerstoffsensor-Signalantwort eine Änderung der Sauerstoffkonzentration nachzuweisen, die auf einen Kohlenmonoxid-Durchbruch hinweist.

2. Sensorsystem nach Anspruch 1, wobei der Nachweis des Kohlenmonoxid-Durchbruchs einen Alarm auslöst.

3. Sensorsystem nach Anspruch 1, wobei der Nachweis des Kohlenmonoxid-Durchbruchs eine Änderung eines aktuellen Verhältnisses von Kraftstoff zu Sauerstoff auslöst, das in den industriellen Prozess eingegeben wird.

4. Sensorsystem nach Anspruch 1 und ferner umfassend:
einen Sender (100), der konfiguriert ist, um einen Hinweis auf den nachgewiesenen Kohlenmonoxid-Durchbruch zu übermitteln.

5. Sensorsystem nach Anspruch 1, wobei der Sauerstoffdetektionssensor (112) eine elektrochemische Zelle auf Zirciumoxidbasis umfasst und wobei die elektrochemische Zelle auf Zirciumoxidbasis eine Spannung an Elektroden misst, die ein Referenzgas von dem Abgasstrom trennen.

6. Sensorsystem nach Anspruch 1, wobei der katalytische Konverter einen Platinkatalysator umfasst.

7. Sensorsystem nach Anspruch 1, wobei der Signaldetektor ferner konfiguriert ist, um eine Kohlenmonoxidkonzentration im Abgasstrom zu berechnen, zumindest teilweise auf Basis der nachgewiesenen Änderung der Sauerstoffkonzentration.

8. Verfahren zum Nachweis von Kohlenmonoxid in einem Abgasstrom eines industriellen Prozesses, wobei das Verfahren umfasst:

Nachweisen einer abrupten Änderung der Sauerstoffkonzentration im Abgasstrom des industriellen Prozesses, wobei die nachgewiesene abrupte Änderung auf einen Kohlenmonoxid-Durchbruch hinweist und wobei der Nachweis durch Einsatz eines Sauerstoffsensors (112) und dessen Sauerstoffsensorsignals vervollständigt wird; **gekennzeichnet durch**

das Berechnen einer Kohlenmonoxidkonzentration im Abgasstrom unter Verwendung der nachgewiesenen Änderung der Sauerstoffkonzentration in einem ersten Erfassungsmodus auf Basis des Sauerstoffsensorsignals und das Berechnen einer Kohlenmonoxidkonzentration im Abgasstrom unter Verwendung der nachgewiesenen Änderung der Sauerstoffkonzentration in einem zweiten Modus auf Basis einer Ableitung einer Sauerstoffsensor-Signalantwort.

9. Verfahren nach Anspruch 8 und ferner umfassend:
Melden der nachgewiesenen Änderung der Sauerstoffkonzentration.

10. Verfahren nach Anspruch 8 und ferner umfassend:
Melden der berechneten Änderung der Kohlenmonoxidkonzentration.

11. Verfahren nach Anspruch 8 und ferner umfassend:
Ändern eines vorhandenen Verhältnisses von Kraftstoff zu Sauerstoff zumindest teilweise basierend auf der nachgewiesenen Konzentration von Kohlenmonoxid zu einem neuen Verhältnis von Kraftstoff zu Sauerstoff.

12. Verfahren nach Anspruch 8 und ferner umfassend:
Übermitteln einer Angabe der berechneten Kohlenmonoxidkonzentration.

13. Verfahren nach Anspruch 8 und ferner umfassend:

Auslösen eines Alarms, der darauf hinweist, dass im Abgasstrom des industriellen Prozesses Kohlenmonoxid vorhanden ist.

14. Verfahren nach Anspruch 8, wobei der Sauerstoffsensor (112) eine elektrochemische Zelle auf Zirconiumoxidbasis umfasst.

**Revendications**

1. Système capteur configuré pour détecter de l'oxygène dans un courant à l'échappement d'un processus industriel, le système capteur comprenant :

   une sonde (104) avec un capteur de détection d'oxygène (112), dans lequel le capteur de détection d'oxygène (112) est configuré pour détecter une concentration d'oxygène dans le courant d'échappement et est configuré pour délivrer un signal correspondant, au moins en partie, à la concentration d'oxygène détectée ;
   un convertisseur catalytique placé sur le capteur (112), dans lequel le convertisseur catalytique est configuré pour convertir du monoxyde de carbone en dioxyde de carbone ; et
   un détecteur de signal, **caractérisé en ce que**
   le détecteur de signal est configuré pour comprendre deux modes de détection, de sorte que le détecteur de signal est configuré dans le premier mode afin de détecter un changement de concentration d'oxygène indicatif d'une irruption de monoxyde de carbone sur la base du signal provenant du capteur de détection d'oxygène, et dans le second mode afin de détecter un changement de concentration d'oxygène indicatif d'une irruption de monoxyde de carbone sur la base d'une dérivée d'une réponse au signal du capteur d'oxygène.

2. Système capteur selon la revendication 1, dans lequel la détection de l'irruption de monoxyde de carbone déclenche une alerte.

3. Système capteur selon la revendication 1, dans lequel la détection de l'irruption de monoxyde de carbone déclenche un changement d'un rapport actuel carburant/oxygène injecté dans le processus industriel.

4. Système capteur selon la revendication 1, comprenant en outre :
   un émetteur (100) configuré pour émettre une indication de l'irruption de monoxyde de carbone détectée.

5. Système capteur selon la revendication 1, dans lequel le capteur de détection d'oxygène (112) comprend une cellule électrochimique à base de zirconium, et dans lequel la cellule électrochimique à base de zirconium mesure une tension aux bornes d'électrodes qui sépare un gaz de référence vis-à-vis du courant à l'échappement.

6. Système capteur selon la revendication 1, dans lequel le convertisseur catalytique comprend un catalyseur au platine.

7. Système capteur selon la revendication 1, dans lequel le détecteur de signal est en outre configuré pour calculer une concentration de monoxyde de carbone dans le courant d'échappement, en se basant au moins en partie sur le changement détecté de la concentration en oxygène.

8. Procédé pour détecter du monoxyde de carbone dans un courant d'échappement d'un processus industriel, le procédé comprenant les étapes consistant à :

   détecter un changement abrupt de concentration d'oxygène dans le courant d'échappement du processus industriel, dans lequel le changement abrupt détecté est indicatif d'une irruption de monoxyde de carbone, et dans lequel la détection est complétée en utilisant un capteur d'oxygène (112) et son signal de capteur d'oxygène ;
   **caractérisé par** l'étape consistant à calculer, dans un premier mode de détection, une concentration de monoxyde de carbone dans le courant d'échappement en utilisant le changement détecté de concentration d'oxygène sur la base du signal du capteur d'oxygène et à calculer, dans un second mode, une concentration de monoxyde de carbone dans le courant d'échappement en utilisant le changement détecté de concentration d'oxygène sur la base d'une dérivée d'une réponse du signal du capteur d'oxygène.

9. Procédé selon la revendication 8, comprenant en outre :
   l'étape consistant à faire un rapport du changement détecté de concentration d'oxygène.

**10.** Procédé selon la revendication 8, comprenant en outre :
l'étape consistant à faire un rapport du changement calculé de concentration de monoxyde de carbone.

**11.** Procédé selon la revendication 8, comprenant en outre :
l'étape consistant à modifier un rapport existant carburant/oxygène au moins en partie sur la base de la concentration détectée de monoxyde de carbone pour donner un nouveau rapport carburant/oxygène.

**12.** Procédé selon la revendication 8, comprenant en outre :
l'étape consistant à transmettre une indication de la concentration calculée de monoxyde de carbone.

**13.** Procédé selon la revendication 8, comprenant en outre :
l'étape consistant à déclencher une alerte indiquant que du monoxyde de carbone est présent à l'intérieur du courant d'échappement du processus industriel.

**14.** Procédé selon la revendication 8, dans lequel le capteur d'oxygène (112) comprend une cellule électrochimique à base de zirconium.

# FIG. 1

FIG. 2

O₂-Sensor Response to Co at 2% O₂

FIG. 3A

Derivative (dE/dt) of O₂ – Sensor
Signal Response to CO at 2% O₂

FIG. 3B

370

Derivative (dE/dt) of O$_2$-sensor signal response to CO at 2% O$_2$

FIG. 3C

O₂ sensor reproducibility to 1000ppm CO at 2% O₂

FIG. 3D

Derivative (dE/dt) of O₂ -sensor signal reproducibility to 1000ppm CO at 2% O₂

FIG. 3E

O₂- sensor response to 2.0% CO at 2% O₂

FIG. 4A

Derivative (dE/dt) of O₂-sensor signal response to
2% CO at 2% O₂

FIG. 4B

Derivative (dE/dt) of $O_2$-sensor signal response to 1% CO at 5% $O_2$

430

FIG. 4C

Derivative (dE/dt) of $O_2$-sensor signal response to 1% CO at 20% $O_2$

440

FIG. 4D

500

```
┌─────────────────────────────────────────────────┐
│            COMBUSTION INITIATED                  │─── 502
└─────────────────────────────────────────────────┘
                        │
                        ▼
┌─────────────────────────────────────────────────┐
│        CARBON MONOXIDE BREAKTHROUGH              │─── 504
└─────────────────────────────────────────────────┘
                        │
                        ▼
┌─────────────────────────────────────────────────┐
│  CARBON MONOXIDE CONVERTED TO CARBON DIOXIDE ON  │─── 506
│              PLATINUM CATALYST                   │
└─────────────────────────────────────────────────┘
                        │
                        ▼
┌─────────────────────────────────────────────────┐
│  DETECTION OF AN OXYGEN CONCENTRATION DROP IN    │─── 508
│    RESPONSE TO CARBON DIOXIDE GENERATION         │
└─────────────────────────────────────────────────┘
                        │
                        ▼
┌─────────────────────────────────────────────────┐
│   REPORT DETECTION OF OXYGEN CONCENTRATION       │─── 510
│                  CHANGE                          │
└─────────────────────────────────────────────────┘
                        ┆
                        ▼
┌─────────────────────────────────────────────────┐
│          FUEL/OXYGEN RATIO ALTERED               │─── 512
└─────────────────────────────────────────────────┘
```

# FIG. 5A

550

DERIVATIVE MODE
INITIATED
560

OXYGEN SENSOR
TURNED ON
552

OXYGEN SIGNAL SPIKE
DETECTED
554

CARBON MONOXIDE
CONCENTRATION
CALCULATED
556

CARBON MONOXIDE
CONCENTRATION
DISPLAYED
558

CHANGE IN FUEL/OXYGEN
RATIO INITIATED
562

# FIG. 5B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 1523214 A

- EP 1327880 A2

**Non-patent literature cited in the description**

- **FOLCH et al.** Solid Electrolyte Multisensor System for Detecting O2, CO, and NO. *Journal Of The Electrochemical Society,* 2007, vol. 1046, J201

- **CAN et al.** Detection of carbon monoxide by using zirconia oxygen sensor. *Solid State Ionics,* 1995, 344-348